# EUROPÄISCHE PATENTANMELDUNG

(11) **EP 2 345 362 A1**
(43) Veröffentlichungstag der Anmeldung: **20.07.2011**
(21) Anmeldenummer: 11150742.2
(22) Anmeldetag: 12.01.2011
(51) Int. Cl.: A61B 3/12, A61B 3/00

(54) **Funduskamera**

(30) Priorität: 18.01.2010 DE 102010004884; 18.01.2010 US 295864 P
(71) Anmelder: Dieter Mann GmbH, 63814 Mainaschaff (DE)
(72) Erfinder: Mann, Dieter, 63839, Kleinwallstadt (DE)
(74) Vertreter: Prüfer & Partner GbR European Patent Attorneys

(57) **Zusammenfassung**

Es wird eine Handkamera zum Fotografieren eines Augenhintergrunds bereitgestellt. Die Kamera besteht aus einem Rohr (3c), einem Kameragehäuse (3a, 3b), das lichtdicht an dem einen Ende (3d) des Rohres angebracht ist, einer Kameravorrichtung (4), die Bilder mittels eines 2-dimensionalen optischen Sensors oder eines fotografischen Films erfassen kann und in dem Kameragehäuse (3a, 3b) untergebracht ist, einem im Strahlengang des vom Augenhintergrund reflektierten Lichts angebrachten Teleobjektiv (5), das in dem Kameragehäuse (3a, 3b) untergebracht ist, einer Weitwinkellinse (2), die im Strahlengang des vom Augenhintergrund reflektierten Lichts an dem anderen Ende (3e) des Rohres (3c) angebracht ist, und einer Lichtzuführeinrichtung (1), die aus einem homogenen translumineszentem Material besteht, wobei die Lichtzuführeinrichtung (1) so bezüglich des Rohres (3c) angeordnet ist, dass bei hinreichender Annäherung des anderen Endes (3e) des Rohres (3c) an das untersuchende Auge die Lichtzuführeinrichtung die Sklera des Auges berührt, falls die Mitte der Weitwinkellinse auf die Mitte der Pupille ausgerichtet ist.

## Beschreibung

Die vorliegende Erfindung bezieht sich auf eine Funduskamera, welche zur Abbildung des Augenhintergrundes (Fundus) dient. Insbesondere bezieht sich die Erfindung auf eine Funduskamera mit kompakten Abmessungen, die ohne Weiteres in der Hand gehalten werden kann.

Der grundsätzliche Aufbau einer Funduskamera besteht aus einem mehrstufigen optischen System, bei dem eine in unmittelbarer Nähe des Auges angeordnete Weitwinkellinse ein Zwischenbild erzeugt, welches von weiteren optischen Komponenten auf einen Film oder eine CCD-Matrix abgebildet wird. Ein Problem, das bei der Beobachtung des Augenhintergrundes stets auftritt, ist dabei die Notwendigkeit, Beleuchtungslicht in das Auge zu bringen.

Üblicherweise lässt man das Beleuchtungslicht durch die Pupille in das Auge eintreten. Hierbei trifft man allerdings auf das Problem, dass es an den brechenden Medien im Auge zu Reflexen kommt, die die Beobachtung stören. US 3,944,341 beschreibt deshalb eine Funduskamera, bei der zwar das Licht durch die Pupille eintritt, jedoch durch Wahl einer ringförmigen Beleuchtung mittels Lichtleitern eine Reflexion an der Linse des Auges dadurch vermieden wird, dass der Randbereich der Pupille für die Beleuchtung verwendet wird. In der Druckschrift werden für die Beleuchtung zwei aufwendig gestaltete Lichtfaserringe verwendet. Da aber diese Art der Beleuchtung aufwendig ist, wird oftmals die nahe dem Auge positionierte Vorsatzlinse auch für die Zuführung des Beleuchtungslichts verwendet, wie es beispielsweise in US 3,936,844 beschrieben ist. Solch eine Ausgestaltung zieht aber wiederum eine kompliziert gestaltete Optik nach sich, da die Strahlengänge der Beleuchtung und Bildgebung nahe beieinander liegen. Die Augenhintergrundbeobachtungsvorrichtung und -kamera werden hierdurch sehr sperrig.

Aufgrund der oben geschilderten Probleme bei transpupillärer Beleuchtung wurde im Stand der Technik, beispielsweise in US 3,954,329, vorgeschlagen, den Augenhintergrund durch die Sklera zu beleuchten. Diese Beleuchtungsweise ist aber bis heute eher unüblich, obwohl die Druckschrift US 3,954,329 bereits im Jahre 1976 veröffentlicht wurde. Dies mag damit zusammenhängen, dass die Lichtstärke für eine Durchleuchtung der Sklera groß sein muss, und aus diesem Grunde die Verwendung eines Lichtfaserringes nicht möglich ist. In Vorrichtungen des Standes der Technik, in denen eine transsklerale Beleuchtung verwendet wird, wird deshalb normalerweise das Licht über ein Faserbündel punktuell durch die Sklera gestrahlt (siehe beispielsweise US 3,943,329). Zur homogenen Ausleuchtung des Augenhintergrundes ist dabei das Durchstrahlen der Sklera an mehr als einer Stelle notwendig. Die deshalb notwendige Anbringung von mehr als zwei Faserbündeln macht die Funduskamera sperrig.

Angesichts der oben geschilderten Probleme ist es eine Aufgabe der vorliegenden Erfindung, eine Funduskamera bereitzustellen, die kompakt ausgestaltet ist, so dass sie frei mit einer Hand gehalten werden kann.

Die Aufgabe wird gelöst durch eine Handkamera nach Anspruch 1. Weiterbildungen der Erfindung sind in den Unteransprüchen angegeben.

Durch die Erfindung wird es möglich, eine mobile Funduskamera mit einem großen Winkelbereich von ca. 100° oder mehr zu schaffen. Die Mobilität der mit einer Hand zu bedienenden Kamera spart Zeit und Kosten, insbesondere wenn Patienten untersucht werden müssen, die in Kliniken außerhalb der augenheilkundlichen Abteilungen behandelt werden. Solch eine mobile Kamera bietet aber insbesondere auch für Frühgeborene einen beachtlichen Vorteil: Frühgeborene lassen sich nur schwer untersuchen, da sie aufgrund des Inkubators nur kostenintensiv und mit erhöhtem Risiko transportiert werden können. Eine Verlagerung eines Frühgeborenen in eine augenheilkundliche Abteilung, in der eine Standfunduskamera benutzt werden kann, gestaltet sich daher als sehr aufwendig. Dieses Problem lässt sich mit der erfindungsgemäßen Kamera lösen.

Im Folgenden werden Ausführungsbeispiele der Erfindung unter Bezugnahme auf die Zeichnungen beschrieben. Von den Figuren zeigen:
Fig. 1 eine erfindungsgemäße Handkamera, bei der während der Aufnahmen die Weitwinkeloptik keinen direkten Kontakt zum Auge hat, und
Fig. 2 eine erfindungsgemäße Handkamera, bei der während der Aufnahmen die Weitwinkeloptik die Hornhaut des Auges berührt.

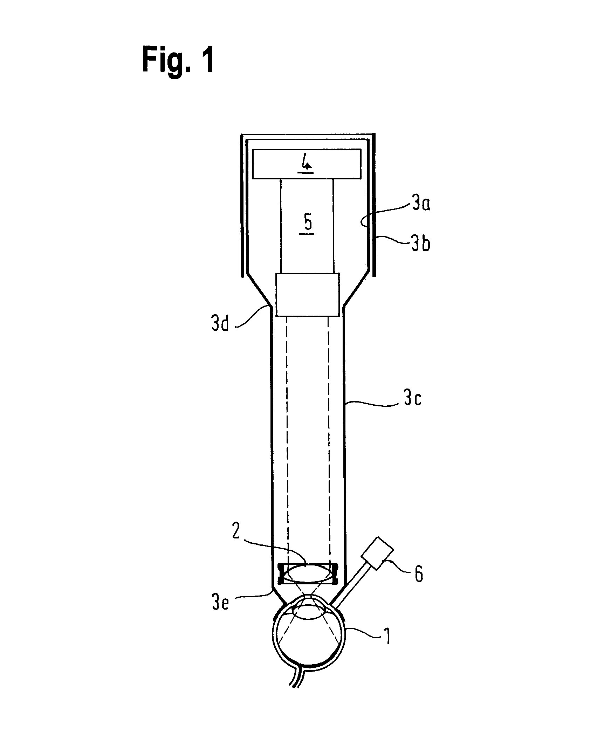

Die erfindungsgemäße Kamera umfasst ein Rohr 3c, an dessen dem zu untersuchenden Auge zugewandten Ende 3e eine Weitwinkellinse 2 angebracht ist. An dem dem zu untersuchenden Auge abgewandten Ende 3d des Rohres sind ein Teleobjektiv 5 und eine Kamera 4 angebracht. Das Teleobjektiv 5 und die Kamera 4 sind von einem Gehäuse 3a, 3b umgeben, welches lichtdicht mit dem Rohr 3c verbunden ist. Wie man in Fig. 1 sieht, ist die verwendete Optik sehr einfach gestaltet. Im Wesentlichen ist der Innenraum des Rohres 3c leer. Das von der Weitwinkellinse 2 erzeugte virtuelle Bild wird von dem Teleobjektiv 5 auf einen zweidimensionalen optischen Sensor, wie beispielsweise einen CCD (charge coupled device)-Sensor oder einen APS-Sensor (aktiver Pixelsensor bzw. CMOS-Sensor) oder aber auf einen fotografischen Film abgebildet.

Bei der Kamera kann es sich um eine kompakte Digitalkamera handeln, die sehr ähnlich einer Digitalkamera ist, welche allgemein im Handel für private Endverbraucher angeboten wird. Dies gilt auch für das Teleobjektiv 5.

Zur Änderung des Fokus kann entweder ein handelsübliches Zoomobjektiv verwendet werden, oder aber es wird ein Kameragehäuse 3a, 3b verwendet, wie es in Fig. 1 dargestellt ist. Fig. 1 zeigt, dass die Kamera mit einem oberen Gehäuseteil 3b verbunden sein kann, welches haubenartig ein mit dem Rohr 3c verbundenes unteres Kameragehäuseteil 3a überdeckt. Das untere Kameragehäuseteil 3a hat dabei die Gestalt eines nach oben offenen Zylinderstutzens. Wird nun das obere Kameragehäuseteil 3b in Richtung der Längsachse des Rohres 3c bezüglich des unteren Kameragehäuseteils 3a bewegt, so kann auf diese Weise der Abstand zwischen dem Teleobjektiv 5 und der Weitwinkellinse 2 verändert werden und dadurch der Fokus verändert werden. Es sei noch bemerkt, dass ein Bediener der Kamera in ähnliches Weise wie bei kommerziell zu erhaltenden Kameras durch einen Sucher oder einen auf der Rückseite der Kamera angebrachten Bildschirm blicken kann, der aus Gründen der Vereinfachung nicht in Fig. 1 dargestellt ist.

Die Beleuchtung des Augenhintergrundes wird bei der vorliegenden Erfindung transskleral durchgeführt. Zu diesem Zweck ist ein zylindrischer Ringvorsatz 1 aus translumineszentem Material am dem Auge zugewandten Ende 3e des Rohres 3c angebracht. Dieser als Lichtzuführeinrichtung dienende Ringvorsatz wird konzentrisch zur Hornhaut limbusnah auf das Auge aufgesetzt, so dass er auf der Sklera außerhalb der Pupille auf dem Auge aufliegt. Von einer Lichtquelle 6 kann dem Ringvorsatz 1 Licht zugeführt werden. Dies kann beispielsweise mittels Lichtleitern geschehen, die an die Seitenwand des Ringvorsatzes 1 angekoppelt sind. Beispielsweise kann aus der Seitenwand des Ringvorsatzes 1 ein Stutzen aus dem translumineszenten Material des Ringvorsatzes 1 ragen. Auf diesem Stutzen ist dann eine geeignete Koppelvorrichtung zum Aufstecken oder Aufschrauben eines die Lichtleitfasern enthaltenden Kabels angebracht.

Da das Auge eine Krümmung aufweist und der Ringvorsatz 1 mit seiner Stirnseite auf dem Auge aufliegt, ist es von Vorteil, wenn die Fläche der Stirnseite nicht einen rechten Winkel zur Längsachse des Rohres 3c aufweist. Beispielsweise kann der Außenrand des Ringes weiter in Richtung des Auges hervorstehen als der Innenrand. Im Idealfall weist die Stirnfläche des Ringvorsatzes zusätzlich eine Krümmung auf, die an die Krümmung eines Durchschnittsauges angepasst ist.

Bei dem Ringvorsatz macht man sich eine Totalreflexion des Lichtes an der Grenzfläche zwischen dem Ringmaterial und der umgebenden Luft für die Lichtleitung zunutze. Das Licht wird so gleichmäßig durch den Ringvorsatz geleuchtet und im Idealfall ringförmig durch die Sklera geleuchtet.

Der Ringvorsatz 1 kann aus translumineszentem Kunststoffmaterial oder aus Glas bestehen. Durch die Verwendung dieses aus homogenem Material ausgebildeten Ringvorsatzes wird gegenüber einem Lichtfaserring eine größere Lichtstärke erhalten. Dies liegt unter anderem daran, dass die Lichtfasern eine viel kleinere Gesamtfläche aufweisen als der Ringvorsatz. Während Lichtleitfasern in der Regel einen Durchmesser von einigen 100 µm aufweisen, kann die Wandstärke bei dem Ringvorsatz einige mm betragen. Durch eine spezielle Wahl des Ringmaterials können weiterhin Verluste bei der Lichtleitung minimiert werden.

Es gibt noch einen weiteren Vorteil der Verwendung solch eines Ringvorsatzes gegenüber der Verwendung von Lichtleiterfasern:

Es ist bekannt, dass sich Lichtleiterfasern wegen der Wärmeleitung des Materials sehr stark erwärmen. Dies kann zu einer Schädigung der Bindegewebszellen am Auge, auf das die Lichtleiterfasern im Stand der Technik aufgesetzt werden, führen, wenn die Temperatur 42 °C übersteigt. Bei der Erfindung wird eine solche Erwärmung bereits durch die größere Aufsetzfläche auf das Auge vermieden. Darüberhinaus kann durch eine gezielte Materialwahl eine zu starke Erwärmung der auf dem Auge aufsitzenden Flächenbereiche des Ringvorsatzes vermieden werden. Beispielsweise kann als Ringmaterial Borosilikatglas 3.3 verwendet werden, welches kurz- und langwellige Anteile der Strahlung blockt. Zur Vermeidung von Schädigungen ist es in jedem Fall günstig, den Durchmesser des Ringvorsatzes 1 so zu wählen, dass er, wenn das Rohr 3c zentrisch zur Pupille ist, an einer Stelle der Sklera aufsetzt, die von möglichst wenig Blutgefäßen durchzogen ist. Beispielsweise könnte der Durchmesser des Ringvorsatzes so gewählt werden, dass der Abstand zwischen Aufsetzstelle des Ringvorsatzes auf der Sklera und Limbus ca. 2mm beträgt.

Mit der erfindungsgemäßen Funduskamera wird eine mobile Handkamera bereitgestellt. Solch eine Kamera kann insbesondere von dem Bediener während der Untersuchung in der Hand gehalten werden, ohne dass ein Kamerastativ verwendet werden muss. Im Idealfall gestaltet sich die Verwendung der Kamera als nicht sehr viel komplizierter als die Verwendung einer herkömmlichen Digitalkamera. Die normalerweise bei transskleraler Beleuchtung auftretende Rotstichigkeit der Bilder kann nach einer Digitalisierung von der Kamerasoftware herausgerechnet werden. Dadurch werden komplizierte Optiken oder unterschiedliche Farbfilter im Beleuchtungsstrahlengang zur Farbkorrektur vermieden.

Die transsklerale Beleuchtung bietet den entscheidenden Vorteil, dass es keine Probleme mit Reflexionen an der Linse gibt. Des Weiteren liegt ein entscheidender Vorteil aber auch darin, dass Untersuchungen selbst bei kleinen Pupillendurchmessern bzw. krankhaften Veränderungen der Linse, die eine transpupilläre Beleuchtung unmöglich machen, möglich sind. Beispielsweise kann die Ausbildung von Tumoren die Beleuchtung durch den Randbereich der Pupille unmöglich machen. Wie bereits eingangs erwähnt wurde, ist die Kamera insbesondere zur Untersuchung von Frühgeborenen geeignet. Um dies zu ermöglichen, kann einfach ein Ringvorsatz 1 mit einem kleineren Durchmesser und einem steileren Radius gewählt werden als jenen, die für Erwachsene vorgesehen sind. Mit "steilerer Radius" ist hierbei Folgendes gemeint: Infolge der größeren Krümmung des kleineren Frühgeborenenauges ist die Stirnfläche des Ringvorsatzes so ausgebildet, dass der Ringaußenrand gegenüber dem Ringinnenrand stärker in Richtung des Auges vorgeschoben ist, als dies bei dem Ringvorsatz für Erwachsene der Fall ist.

Schließlich ermöglicht es die transsklerale Beleuchtung, den gesamten Fundus zu beleuchten, was bei einer transpupillären Beleuchtung infolge des begrenzten Pupillendurchmessers so nicht möglich ist. Damit sind, bei entsprechender Wahl der Weitwinkellinse 2, Bildwinkel bis zu 165° möglich.

### Weitere Ausgestaltungen der Erfindung

Es wurde im Zusammenhang mit Fig. 1 ein Ringvorsatz 1 beschrieben, der vollständig die Pupille umgibt. Der Grund für solch ein vollständiges Umschließen der Pupille ist eine möglichst gleichmäßige Zuführung des Beleuchtungslichtes. Natürlich kann das Beleuchtungslicht aber auch an mehreren von einander getrennten Stellen rund um die Pupille herum durch die Sklera gestrahlt werden. Beispielsweise kann die Stirnfläche des Ringvorsatzes, die auf das Auge aufgesetzt wird, Ausnehmungen aufweisen. Diese Ausnehmungen können sich im Extremfall sogar über den größten Teil der Wandfläche des Ringvorsatzes ausdehnen. Der Ringvorsatz 1 kann dadurch beispielsweise an sechs, fünf, vier, drei oder gar nur zwei Stellen auf der Sklera aufsitzen. Natürlich sollten die Stellen, an denen der Ringvorsatz 1 die Sklera berührt, vorzugsweise in Umfangsrichtung mit gleichem Abstand zueinander rund um die Pupille herum angeordnet sein.

Natürlich ist es auch möglich, anstelle einer Weitwinkellinse 2 ein Linsensystem oder eine Optik mit weiteren Elementen vorzusehen. Je einfacher solch eine Weitwinkeloptik 2 gestaltet ist und je weniger Bestandteile sie aufweist, umso geringer ist jedoch das Gewicht der Handkamera. Der Begriff "Weitwinkeloptik" soll hier auch Optiken umfassen, die nur aus einer einzigen Weitwinkellinse bestehen.

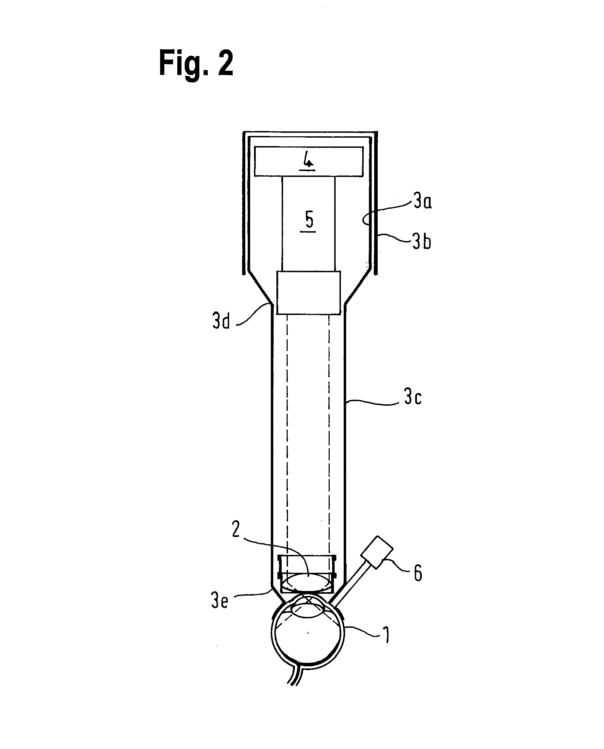

Die am dem Auge zugewandten Ende 3e des Rohres 3c angeordnete Weitwinkeloptik 2 kann in Bezug auf die auf das Auge aufzusetzende Stirnfläche des Ringvorsatzes 1 so angeordnet sein, dass gleichzeitig mit dem Aufsetzen des Ringvorsatzes auf die Sklera die Weitwinkeloptik 2 auf die Hornhaut des Auges aufgesetzt wird (indirekte Ophthalmoskopie, siehe Fig. 2). Natürlich kann aber auch die Weitwinkeloptik mit einem bestimmten Abstand zu dem dem Auge zugewandten Ende 3e des Rohres 3c in dem Rohr 3c angeordnet sein (siehe Fig. 1). Im Idealfall ist die Weitwinkeloptik 2 entlang der Längsachse des Rohres 3c verstellbar.

Bei einer weiteren Ausgestaltung der Erfindung wird auf den Ringvorsatz 1 verzichtet, und das Beleuchtungslicht wird über einen Stab aus translumineszentem homogenem Material zugeführt, wobei der Stab auf eine Stelle der Sklera aufgesetzt wird. Durch die hohe Lichtstärke infolge der Verwendung solch eines Lichtstabes kann für eine ausreichende Beleuchtung des Augenhintergrundes gesorgt werden, obwohl nur punktuell die Sklera durchstrahlt wird. Im Übrigen kann der Lichtstab aus demselben Material bestehen wie der Ringvorsatz 1. Zu einer Verbesserung der Lichtleitfähigkeit können sowohl der Ringvorsatz 1 als auch der Lichtstab mit einem Licht reflektierenden Material ummantelt sein, beispielsweise mit einer Silberschicht oder einem Metallrohr überzogen sein.

Bei einer Variante der Erfindung sind der Lichtstab oder der Ringvorsatz unabhängig von dem Rohr 3c bewegbar. In diesem Fall werden nur der Lichtstab oder der Ringvorsatz auf das Auge aufgesetzt, während das Rohr 3c mit der Weitwinkeloptik in einem bestimmten Abstand zum Auge gehalten wird. Hier kann es von Vorteil sein, wenn die Kamera auf einem Kreuzschlitten montiert ist, da eine Hand für die Positionierung des Lichtstabes oder Ringvorsatzes benötigt wird.

Allgemein ist es von Vorteil, wenn die Handkamera auch auf einen Kreuzschlitten montierbar ist. Ist der Kreuzschlitten auf einen höhenverstellbaren Tisch montiert, so erhält man durch die Befestigung der Handkamera am Kreuzschlitten ein Standgerät, mit dem Aufnahmen an sitzenden Patienten durchgeführt werden können. Gerade bei Tumorpatienten ist es beispielsweise wichtig, dass Aufnahmen sowohl am sitzenden als auch am liegenden Patienten durchgeführt werden. Die Aufnahmen im liegenden Zustand werden dann mit der nicht an einen Kreuzschlitten montierten Handkamera gemacht. Weiterhin ist es möglich, die Kamera über ein Kabel mit einem Monitor zu verbinden, so dass man sich das Kamerabild auf dem Monitor anschauen kann.

Es sei noch darauf hingewiesen, dass die Verwendung des Begriffes "Zylinder" in der vorliegenden Anmeldung nicht auf Kreiszylinder beschränkt ist. Vielmehr wird der Begriff "Zylinder" im mathematischen Sinne verwendet und umfasst allgemein Körper, die von zwei parallelen ebenen Flächen beliebiger Gestalt und einer diese Flächen verbindenden Mantelfläche begrenzt werden. Insbesondere soll der Begriff aber gerade Zylinder bezeichnen, bei denen die Mantelfläche auf der Grund- und Deckfläche senkrecht steht.

## Patentansprüche

1. Handkamera zum Fotografieren eines Augenhintergrunds mit:
einem Rohr (3c)
einem Kameragehäuse (3a, 3b), das lichtdicht an dem einen Ende (3d) des Rohres angebracht ist,
einer Kameravorrichtung (4), die Bilder mittels eines 2-dimensionalen optischen Sensors oder eines fotografischen Films erfassen kann und in dem Kameragehäuse (3a, 3b) untergebracht ist,
einem im Strahlengang des vom Augenhintergrund reflektierten Lichts angebrachten Teleobjektiv (5), das in dem Kameragehäuse (3a, 3b) untergebracht ist
einer Weitwinkeloptik (2), die im Strahlengang des vom Augenhintergrund reflektierten Lichts an dem anderen Ende (3e) des Rohres (3c) angebracht ist, und
einer Lichtzuführeinrichtung (1), die aus einem homogenen translumineszentem Material besteht, wobei die Lichtzuführeinrichtung (1) geeignet ist, auf die Sklera des zu untersuchenden Auges aufgesetzt zu werden zur transskleralen Beleuchtung des Augenhintergrundes.

2. Handkamera nach Anspruch 1, wobei die Lichtzuführeinrichtung (1) so bezüglich des Rohres (3c) angeordnet ist, dass bei hinreichender Annäherung des anderen Endes (3e) des Rohres (3c) an das untersuchende Auge die Lichtzuführeinrichtung die Sklera des Auges berührt, falls die Mitte der Weitwinkeloptik auf die Mitte der Pupille ausgerichtet ist.

3. Handkamera nach Anspruch 1 oder 2, bei der die Lichtzuführeinrichtung (1) ein Lichtstab ist.

4. Handkamera nach Anspruch 1 oder 2, bei der die Lichtzuführeinrichtung (1) ein hohlzylinderförmiger Ringvorsatz ist, der an dem anderen Ende (3e) des Rohres (3c) so angebracht ist, dass er mit seiner Stirnfläche konzentrisch zur Pupille auf die Sklera des Auges aufgesetzt werden kann.

5. Handkamera nach Anspruch 4, bei der der Ringvorsatz (1) an seiner auf die Sklera aufzusetzenden Stirnfläche mindestens eine Ausnehmung aufweist, die so ausgebildet sind, dass die Stirnfläche sich aus einer Mehrzahl von Teilflächen zusammensetzt.

6. Handkamera nach Anspruch 5, bei der die mindestens eine Ausnehmung der Stirnfläche so ausgebildet ist, dass die Stirnfläche sich aus einer Mehrzahl von einzelnen Teilflächen zusammensetzt, die voneinander beabstandet sind.

7. Handkamera nach einem der Ansprüche 1 bis 6, bei der das homogene, translumineszente Material, aus dem die Lichtzuführeinrichtung (1) besteht, Glas oder ein Kunststoff ist.

8. Handkamera nach Anspruch 7, bei der das homogene, translumineszente Material, aus dem die Lichtzuführeinrichtung (1) besteht, Borosilikatglas 3.3 ist.

9. Handkamera nach einem der Ansprüche 1 bis 8, bei der die Lichtzuführeinrichtung (1) mit einer nach innen reflektierenden Schicht ummantelt ist.

10. Handkamera nach einem der Ansprüche 1 bis 9, bei der die Weitwinkeloptik (2) so angeordnet ist, dass sie beim Aufsetzen der Lichtzuführeinrichtung (1) auf die Sklera genau dann die Hornhaut des Auges berührt, wenn die Lichtzuführeinrichtung (1) die Sklera berührt.

11. Handkamera nach einem der Ansprüche 1 bis 10, bei der die Weitwinkeloptik (2) innerhalb des Rohres (3c) angeordnet ist und ihre Position im Rohr entlang der Längsachse des Rohres variiert werden kann.
